# EUROPEAN PATENT APPLICATION

(11) **EP 4 834 353 A1**
(43) Date of publication of application: **04.11.2026**
(21) Application number: 24913478.4
(22) Date of filing: 28.11.2024
(51) Int. Cl.: A61K 31/785, A61K 9/20

(54) **PREPARATION COMPRISING SEVELAMER OR PHARMACEUTICALLY ACCEPTABLE SALT THEREOF AND METHOD FOR PREPARING SAME**

(30) Priority: 27.12.2023 KR 20230192195
(71) Applicant: Daewoong Pharmaceutical Co., Ltd., Hwaseong-si, Gyenoggi-do 18623 (KR)
(72) Inventor: LEE, Jaewon, Yongin-si, Gyeonggi-do 17028 (KR); YANG, Heesang, Yongin-si, Gyeonggi-do 17028 (KR); CHUN, Kyeunghwa, Yongin-si, Gyeonggi-do 17028 (KR); KIM, Gwanyoung, Yongin-si, Gyeonggi-do 17028 (KR)
(74) Representative: Stolmár & Partner Patentanwälte PartG mbB
(86) International application number: PCT/KR2024/019076
(87) International publication number: WO 2025/143585

(57) **Abstract**

The present invention relates to an oral formulation comprising sevelamer or a pharmaceutically acceptable salt thereof, and a method for preparing the same. Since the formulation is prepared through fluidized bed granulation, damage to the formulation is reduced and variation caused by abrasion is decreased, such that superior quality can be maintained. In particular, the formulation of the present invention has a smaller granule size while exhibiting increased uniformity, thereby improving medication convenience through reduction in the size of the formulation.

## Description

### TECHNICAL FIELD

The present invention relates to an oral formulation comprising sevelamer or a pharmaceutically acceptable salt thereof, and to a method for preparing the same, wherein the formulation is prepared through fluidized bed granulation so that damage to the formulation is reduced, variation caused by abrasion is reduced, and excellent quality can be maintained.

### BACKGROUND ART

In patients with renal dysfunction, the amount of phosphorus excreted from the body through the kidneys decreases, and this is difficult to remove even through dialysis. If phosphate is absorbed into the human body as it is, hyperphosphatemia develops, and calcium binds to phosphate, resulting in hypocalcemia in which the blood calcium level decreases. In addition, calcium and phosphate may cause calcification that forms crystals in body tissues including within blood vessel walls, which may lead to severe arteriosclerosis, stroke, heart attack, and circulatory failure.

Sevelamer is a non-calcium-based phosphate-regulating agent that binds to phosphate ingested through food in the gastrointestinal tract and prevents phosphorus from being absorbed into the bloodstream. Sevelamer carbonate is insoluble in water, but tends to swell and form sticky lumps upon contact with water. Due to this swelling tendency, during wet granulation, depending on the conditions, the granules swell and form agglomerated lumps, resulting in differences in physical properties and causing problems in granule uniformity. In addition, because of swelling and lump formation of the granules, the size of tablets increases, making oral administration difficult and thereby causing problems in medication convenience.

Accordingly, there has been a continuous need for research and development of a formulation and a method for preparing the same that can improve medication convenience while maintaining the quality of a sevelamer formulation.

### DESCRIPTION OF EMBODIMENTS

### TECHNICAL PROBLEM

The technical problem to be solved by the present invention is to provide a pharmaceutical composition comprising sevelamer or a pharmaceutically acceptable salt thereof and a pharmaceutically acceptable additive, and comprising granules obtained through fluidized bed granulation.

Another technical problem to be solved by the present invention is to provide a method for preparing an oral formulation, comprising granulating sevelamer or a pharmaceutically acceptable salt thereof through fluidized bed granulation.

### TECHNICAL SOLUTION

One aspect of the present invention for achieving the above object relates to a pharmaceutical composition comprising: sevelamer or a pharmaceutically acceptable salt thereof; and one or more pharmaceutically acceptable additives selected from the group consisting of a binder, a disintegrant, and a lubricant, wherein the pharmaceutical composition comprises granules obtained through fluidized bed granulation.

In one embodiment, the pharmaceutically acceptable salt may be a carbonate, but is not limited thereto, and salts, hydrates, solvates, and the like prepared according to conventional methods in the art may be modified and applied as needed.

In one embodiment, the pharmaceutically acceptable additive may include microcrystalline cellulose, sodium chloride, and zinc stearate, but is not limited thereto.

In the present invention, the term "fluidized bed granulation" refers to a process of agglomerating particles using a fluidized bed granulator, in which a powder sample is placed in an enclosed space (chamber), and while the powder is blown with hot air and subjected to agitation and circulation, a binding liquid is sprayed so that the sample is granulated.

Specifically, in the fluidized bed granulation, the binder may be added in an amount of 0.1 to 5.0 parts by weight based on the total parts by weight of the tablet core, and more specifically, in an amount of 0.5 to 4.5 parts by weight, but is not limited thereto. As used herein, the term "total parts by weight of the tablet core" includes all additives used in the preparation of the tablet core, including the active ingredient and the binding solution.

In one embodiment, the binder may be water, but is not limited thereto, and any aqueous solvent capable of exhibiting the effects of the present invention may be modified and applied as needed.

In one embodiment, the granules obtained through the fluidized bed granulation may have an average particle size of 75 to 400 µm, specifically 100 to 400 µm.

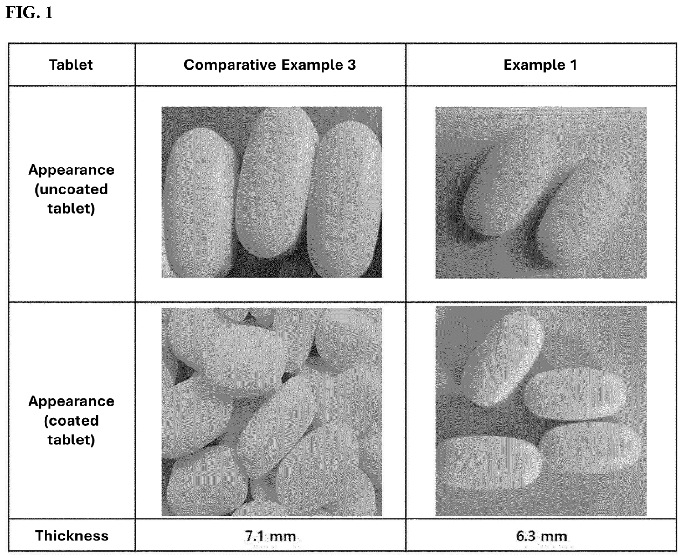

In one example of the present invention, it was confirmed that, under the same composition, when the formulation was prepared by fluidized bed granulation, damage to the tablets was reduced and variation caused by tablet abrasion was decreased, so that superior quality could be maintained (Table 2 and Fig. 1). Furthermore, it was confirmed that the granules had a smaller size while exhibiting increased uniformity (Table 3). Therefore, the pharmaceutical composition of the present invention can maintain uniform quality such that damage to the formulation is minimized through fluidized bed granulation, thereby preventing loss of pharmacological efficacy due to deterioration in formulation quality, while also improving medication convenience due to the reduced size.

The pharmaceutical composition of the present invention may be formulated into an oral dosage form such as a tablet, pellet, capsule, granule, or powder, but is not limited thereto. Specifically, the oral dosage form may be in the form of a tablet.

Another aspect of the present invention relates to a method for preparing an oral formulation, comprising granulating sevelamer or a pharmaceutically acceptable salt thereof through fluidized bed granulation.

In one embodiment, the sevelamer or a pharmaceutically acceptable salt thereof may further comprise one or more pharmaceutically acceptable additives selected from the group consisting of an excipient, a binder, a disintegrant, and a lubricant.

In one embodiment, the binder may be added in an amount of 0.5 to 4.5 parts by weight based on the total parts by weight of the tablet core, and specifically, the binder may be added in an amount of 0.5 to 4.5 parts by weight, but is not limited thereto.

In one embodiment, the method for preparing the oral formulation may comprise: a) mixing sevelamer or a pharmaceutically acceptable salt thereof with an excipient; b) placing the mixture prepared in step a) into a fluidized bed granulator and injecting a binding solution to perform granulation; and c) post-blending sodium chloride and zinc stearate.

The oral formulation prepared by the above method may be in the form of a tablet, pellet, capsule, granule, or powder, and specifically, may be in the form of a tablet.

### ADVANTAGEOUS EFFECTS OF INVENTION

The present invention relates to preparing a formulation comprising sevelamer through fluidized bed granulation, whereby the formulation prepared thereby exhibits reduced damage and less variation due to abrasion, such that superior quality can be maintained. In particular, the formulation of the present invention has a smaller granule size while exhibiting increased uniformity, thereby improving medication convenience through reduction in the size of the formulation.

### BRIEF DESCRIPTION OF THE DRAWINGS

FIG. 1 shows the results of evaluating the abrasion and thickness of tablets prepared according to Comparative Example 3 and Example 1.

### DETAILED DESCRIPTION OF EXEMPLARY EMBODIMENTS

Hereinafter, the present invention will be described in detail through the Examples. However, the following Examples are only for exemplifying the present invention, and the present invention is not limited by the following Examples.

### Preparation Example. Preparation of Sevelamer Carbonate

### 1-1. Preparation of Comparative Examples 1 to 5

According to the composition shown in Table 1 below, sevelamer carbonate and microcrystalline cellulose were mixed and placed in the chamber of a high-speed mixer (PTK, PM-C003), and purified water was injected as a binding solution while operating the agitator at 200 rpm and the chopper at 3,000 rpm, followed by premixing, granulation, and drying. Thereafter, sodium chloride and zinc stearate were post-blended, and after sizing, the resulting mixture was compressed using a rotary tablet press at a hardness of 25 kN, followed by coating, to prepare the final product.

### 1-2. Preparation of Examples 1 to 5 by Fluidized Bed Granulation

According to the composition shown in Table 1 below, sevelamer carbonate and microcrystalline cellulose were mixed and placed in the chamber of a fluidized bed granulator (Bosch, Hüttlin Solidlab 2). Purified water was then injected as a binding solution and sprayed at a rate of 10 to 15 g/min under conditions of an exhaust temperature of 60°C and a spray pressure of 1.0 bar, followed by granulation and drying. Thereafter, sodium chloride and zinc stearate were post-blended, and after sizing, the resulting mixture was compressed into tablets using a rotary tablet press at a hardness of 25 kN, followed by coating, to prepare the final product.

**[Table 1]**

| **Example** | **Preparation Method** | **Sevelamer carbonate (mg)** | **Microcryst alline cellulose (mg)** | **Sodium chloride (mg)** | **Zinc stearate (mg)** | **Purified water (mg)** | **Total (tablet core) (mg)** |
|---|---|---|---|---|---|---|---|
| Comparative Example 1 | High shear granulation (HSM) | 800 | 107.7 | 3.2 | 2.1 | 200 | 913.0 |
| Comparative Example 2 | | 800 | 107.7 | 3.2 | 2.1 | 300 | 913.0 |
| Comparative Example 3 | | 800 | 107.7 | 3.2 | 2.1 | 500 | 913.0 |
| Comparative Example 4 | | 800 | 107.7 | 3.2 | 2.1 | 1,000 | 913.0 |
| Comparative Example 5 | | 800 | 107.7 | 3.2 | 2.1 | 1,500 | 913.0 |
| Example 1 | Fluidized bed granulation | 800 | 107.7 | 3.2 | 2.1 | 500 | 913.0 |
| Example 2 | | 800 | 107.7 | 3.2 | 2.1 | 1,000 | 913.0 |
| Example 3 | | 800 | 107.7 | 3.2 | 2.1 | 2,000 | 913.0 |
| Example 4 | | 800 | 107.7 | 3.2 | 2.1 | 3,000 | 913.0 |
| Example 5 | | 800 | 107.7 | 3.2 | 2.1 | 4,000 | 913.0 |

### Experimental Example 1. Evaluation of Tablet Properties

In order to evaluate the physical properties of Comparative Examples 1 to 5 and Examples 1 to 5 prepared in the above Preparation Example, the hardness, friability, and surface appearance of the tablets were examined. The hardness of the tablets was measured using a hardness tester, and the friability and surface appearance of the tablets were evaluated using a friability tester.

**[Table 2]**

| | Comparative Example 1 | Comparative Example 2 | Comparative Example 3 | Comparative Example 4 | Comparative Example 5 |
|---|---|---|---|---|---|
| Hardness (kp) (compressed at 25 kN) | 10-13 | 12-15 | 10-13 | 10-13 | 15-20 |
| Friability (%) | 0.2 | 0.15 | 0.22 | 0.19 | 0.14 |
| Surface appearance | Easily friable | Easily friable | Easily friable | Easily friable | Easily friable |

| | Example 1 | Example 2 | Example 3 | Example 4 | Example 5 |
|---|---|---|---|---|---|
| Hardness (kp) (compressed at 25 kN) | 10-13 | 15-20 | 15-20 | 20-26 | 30-35 |
| Friability (%) | 0.09 | 0.09 | 0.06 | 0.07 | 0.01 or less |
| Surface appearance | Slightly friable | Slightly friable | Slightly friable | Almost not friable | Not friable |

As shown in Table 2 above, it was confirmed that Examples 1 to 5 prepared by fluidized bed granulation exhibited higher hardness and significantly lower friability than Comparative Examples 1 to 5. In addition, with respect to the surface appearance of the prepared tablets, it was confirmed that Examples 1 to 5 were slightly friable, almost not friable, or not friable, whereas Comparative Examples 1 to 5 were easily friable.

In addition, the appearance was evaluated based on the same amount of binding solution (500 mg/T). As shown in FIG. 1, when Comparative Example 3 and Example 1 were compared, it was confirmed that, in the case of Example 1 prepared by fluidized bed granulation, the thickness was smaller than that of Comparative Example 3, while friability was lower in both the tablet core and the coated tablet. These results indicate that, in the case of tablets prepared using the fluidized bed granulation of the present invention, damage to the tablets is reduced and variation caused by tablet abrasion is decreased, so that superior quality can be maintained.

### Experimental Example 2. Evaluation of Tablet Weight and Size

The weight and size of the tablets were measured and compared with those of existing commercially available products.

As a result, the tablets of Examples 1 to 5 prepared using the fluidized bed granulation of the present invention exhibited an average weight of about 946 mg, whereas existing commercially available sevelamer carbonate tablets exhibited a weight of about 1,000 to 1,150 mg. Accordingly, it was confirmed that the tablets of Examples 1 to 5 prepared using the fluidized bed granulation of the present invention exhibited a weight that was 5 to 10% (w/w) lower than that of the existing commercially available products.

In addition, it was confirmed that the tablets of Examples 1 to 5 prepared using the fluidized bed granulation of the present invention had an average major axis of about 17.4 mm, thereby forming tablets having a diameter about 15% smaller than the major axis of existing commercially available products, which is about 20 mm.

### Experimental Example 3. Evaluation of Granule Size Distribution in Tablets

The granule size distribution of the tablets of Comparative Example 3 and Example 1, prepared using the same amount of binding solution (500 mg/T), was analyzed. Specifically, the particle size distribution was measured using a particle powder analyzer (Bettersizer, PowerPro A1).

**[Table 3]**

| Granule Size Distribution (µm) | Comparative Example 3 (%) | Example 1 (%) |
|---|---|---|
| 0-45 | 7.6 | 0.1 |
| 45-75 | 14.6 | 0.8 |
| 75-150 | 15.8 | 8.4 |
| 150-250 | 4.7 | 64.4 |
| 250-355 | 3.2 | 23.2 |
| 355-425 | 1.4 | 1.3 |
| 425-1,000 | 27.5 | 1.8 |
| >1,000 | 25.2 | 0 |
| Total | 100 | 100 |

As a result, as shown in Table 3 above, in the case of Example 1 prepared by fluidized bed granulation, 87.6% of the granules were distributed within a particle size range of 150 to 355 µm, confirming that the granule size distribution was highly uniform. In addition, in Example 1, granules having a size exceeding 355 µm accounted for only 3.1%, and no granules having a size exceeding 1,000 µm were observed. In contrast, in Comparative Example 3, granules having a size exceeding 355 µm accounted for 54.1%, corresponding to more than half of the total granules, and in particular, granules having a size exceeding 1,000 µm also accounted for 25.2%.

These results indicate that, even when prepared using the same amount of binding solution and the same combination of components, the particle size and uniformity of the resulting granules vary depending on the granulation method. In the present invention, it was confirmed that fluidized bed granulation provides granules having a smaller size while exhibiting increased uniformity.

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described embodiments are only exemplary in all aspects and are not restrictive. For example, each constituent element which is described as a singular form may be implemented in a distributed form, and similarly, constituent elements which are described as being distributed may be implemented in a combined form.

The scope of the present invention is represented by the claims to be described below, and it should be interpreted that the meaning and scope of the claims and all the changes or modified forms derived from the equivalent concepts thereof fall within the scope of the present invention.

## Claims

1. A pharmaceutical composition comprising:
sevelamer or a pharmaceutically acceptable salt thereof; and
one or more pharmaceutically acceptable additives selected from the group consisting of a binder, a disintegrant, and a lubricant,
wherein the pharmaceutical composition comprises granules obtained through fluidized bed granulation.

2. The pharmaceutical composition according to claim 1, wherein the pharmaceutically acceptable salt is a carbonate.

3. The pharmaceutical composition according to claim 1, comprising, as the pharmaceutically acceptable additive, microcrystalline cellulose, sodium chloride, and zinc stearate.

4. The pharmaceutical composition according to claim 1, wherein, in the fluidized bed granulation, the binder is added in an amount of 0.1 to 5.0 parts by weight based on the total parts by weight of the tablet core.

5. The pharmaceutical composition according to claim 3, wherein the binder is water.

6. The pharmaceutical composition according to claim 1, wherein the granules have an average particle diameter of 75 to 400 µm.

7. The pharmaceutical composition according to claim 1, wherein the pharmaceutical composition is in the form of a tablet.

8. A method for preparing an oral formulation, comprising granulating sevelamer or a pharmaceutically acceptable salt thereof through fluidized bed granulation.

9. The method for preparing an oral formulation according to claim 8, further comprising one or more pharmaceutically acceptable additives selected from the group consisting of an excipient, a binder, a disintegrant, and a lubricant in the sevelamer or the pharmaceutically acceptable salt thereof.

10. The method for preparing an oral formulation according to claim 9, wherein the binder is added in an amount of 0.5 to 4.5 parts by weight based on the total parts by weight of the tablet core.

11. The method for preparing an oral formulation according to claim 8, comprising:
a) mixing sevelamer or a pharmaceutically acceptable salt thereof with an excipient;
b) placing the mixture prepared in step a) into a fluidized bed granulator and injecting a binding solution to perform granulation; and
c) post-blending sodium chloride and zinc stearate.

12. The method according to any one of claims 8 to 11, wherein the oral formulation is in the form of a tablet.
